# EUROPÄISCHE PATENTSCHRIFT

(11) **EP 0 415 202 B1**
(45) Veröffentlichungstag und Bekanntmachung des Hinweises auf die Patenterteilung: **01.06.1994**
(21) Anmeldenummer: 90115761.0
(22) Anmeldetag: 17.08.1990
(51) Int. Cl.: C07C 29/141, C07C 31/20

(54) **Verfahren zur Herstellung niederer, mehrwertiger Alkohole**
Method for the production of lower polyhydric alcohols
Procédé pour la fabrication d'alcools polyhydroxylés inférieurs

(30) Priorität: 26.08.1989 DE 3928285
(43) Veröffentlichungstag der Anmeldung: 06.03.1991
(73) Patentinhaber: BASF Aktiengesellschaft, 67063 Ludwigshafen (DE)
(72) Erfinder: Schuster, Ludwig, Dr., D-6703 Limburgerhof (DE)

(56) Entgegenhaltungen:
- EP-A- 0 224 872
- EP-A- 0 269 888
- US-A- 3 691 100
- CHEMICAL ABSTRACTS, Band 72, Nr. 26, 29. Juni 1970, Seite 109, Spalte 1, Zusammenfassung Nr. 134408j, Columbus, Ohio, US; M.YAMAGUCHI: "Hydrogenation of sugars in the presence of solid acid"

## Beschreibung

Die vorliegende Erfindung betrifft ein Verfahren zur Herstellung niederer, mehrwertiger Alkohole durch die katalytische Hydrierung wäßriger Saccharoselösungen bei erhöhter Temperatur und bei erhöhtem Druck und mittels eines Katalysators, dessen katalytisch aktive Masse, im wesentlichen die Metalle Cobalt, Kupfer und Mangan in Mengen von, berechnet auf den auf 100 % normierten Gesamtgehalt an diesen Metallen,
0 bis 100 Gew.% Cobalt
0 bis 85 Gew.% Kupfer und
0 bis 80 Gew.% Mangan
enthält, wobei diese katalytisch aktive Masse von den genannten Metallen entweder nur Cobalt oder mindestens zwei der genannten Metalle enthält und dem außerdem noch anorganische Polysäuren und/oder Heteropolysäuren zugesetzt sein können.

An der Nutzung des in großen Mengen und preiswert verfügbaren, nachwachsenden Rohstoffs Saccharose zur Gewinnung von Polyolen besteht schon seit längerer Zeit Interesse.

So ist es bekannt (Ind. Eng. Chem. Prod. Res. Dev. 9, 210-212 (1970)), Saccharoselösungen in Methanol-Wasser-Gemischen mittels eines CuO-CeO₂-SiO₂-Katalysators zu hydrieren. Die Hydrierung wird in Gegenwart von, bezogen auf Saccharose, 2 Gew.-% Calciumhydroxid ausgeführt. Dabei entsteht ein Produktgemisch, das aus ca. 31 Gew.% Glycerin, 16 Gew.% Ethylenglycol, 18 Gew.% 1,2-Propylenglykol, 16 Gew.% Hexiten und 19 Gew.% anderen Produkten besteht. Dieses Verfahren ist jedoch nach den Angaben seiner Autoren nicht wirtschaftlich.

Natta et al (Chem. Ber. 76, 641-656 (1943)) führen die Hydrierung von Saccharose in alkoholischer Lösung mittels Kupferchromit/Bariumchromit-Katalysatoren aus. Dabei wird eine Ausbeute von bis zu 54 % an Propylenglykol und 14 % an Glycerin, bezogen auf die eingesetzte Saccharose erzielt. Über die Hydrierung von Saccharose in wäßriger Lösung mit diesem Katalysatorsystem gibt es keine Angaben.

Nach Weidenhagen et al (Chem. Ber. 71, 2712-2716 (1938)) läßt sich bei der Saccharosehydrierung die Bildung von Glycerin zugunsten von 1,2-Propylenglycol unterdrücken, wenn ein zweistufiges Hydrierverfahren angewandt wird. Dabei wird in neutraler, wäßriger Lösung die Saccharose zunächst mittels eines Nickel-Molybdän-Katalysators zu Acetol hydriert, das gebildete Acetol abgetrennt und anschließend in einer mittels Calciumhydroxid alkalisierten, wäßrigen Lösung an einem Nickel-Chrom-Katalysator zu 1,2-Propylenglykol hydriert. Nachteilig an diesem Verfahren ist die aufwendige zweistufige Reaktionsführung.

Der vorliegenden Erfindung lag die Aufgabe zugrunde, ein wirtschaftliches Verfahren zur Hydrierung von Saccharose zu Diolen, Triolen und/oder Tetrolen zu finden. Das Verfahren sollte einstufig durchführbar sein und die Möglichkeit bieten, durch einfache Änderung der Reaktionsbedingungen die Zusammensetzung des bei der Hydrierung entstehenden Produktgemisches so zu verändern, daß es je nach Wunsch überwiegend Diole und Triole oder Triole und Tetrole, insbesondere das ansonsten nur als Nebenprodukt vorkommende 1,2,5,6-Hexantetrol, als Hauptprodukt enthält. Zu diesem Zweck sollte ein wiederverwendbares Katalysatorsystem gefunden werden, das sich hinsichtlich seiner Selektivität langzeitstabil verhält.

Dementsprechend wurde ein Verfahren zur Herstellung niederer, mehrwertiger Alkohole durch die katalytische Hydrierung wäßriger Saccharoselösungen bei erhöhter Temperatur und bei erhöhtem Druck und mittels eines Katalysators, dessen katalytisch aktive Masse, im wesentlichen die Metalle Cobalt, Kupfer und Mangan in Mengen von, berechnet auf den auf 100 % normierten Gesamtgehalt an diesen Metallen,
0 bis 100 Gew.% Cobalt
0 bis 85 Gew.% Kupfer und
0 bis 80 Gew.% Mangan
enthält, wobei diese katalytisch aktive Masse von den genannten Metallen entweder nur Cobalt oder mindestens zwei der genannten Metalle enthält und dem außerdem noch anorganische Polysäuren und/oder Heteropolysäuren zugesetzt sein können, gefunden, das dadurch gekennzeichnet ist, daß man der zu hydrierenden Saccharoselösung 0,01 bis 5 Gew.-% einer oder mehrerer basisch wirkender Metallverbindungen der Metalle der ersten und zweiten Hauptgruppe sowie der Gruppe III A des Periodensystems zusetzt.

Somit können die im erfindungsgemäßen Verfahren verwendbaren Katalysatoren beispielsweise alle drei dieser katalytisch aktiven Metallkomponenten enthalten oder nur aus zwei dieser Metallkomponenten hergestellt sein oder aber auch nur aus der Metallkomponente Cobalt bestehen. Vorteilhaft finden jedoch Katalysatoren Verwendung, welche alle drei Komponenten enthalten, wobei solche Katalysatoren bevorzugt sind, welche mindestens einen Mangananteil von 5 Gew.-%, bezogen auf die Gesamtmenge an den katalytisch aktiven Metallkomponenten, berechnet als Metalle, enthalten. Als Zusammensetzungen für eine Reihe von für das vorliegende Verfahren gut geeigneten Katalysatoren seien die in den folgenden Gruppen A bis C aufgeführten Zusammensetzungen beispielhaft genannt:
- A:: 5 bis 90 Gew.% Cobalt
5 bis 80 Gew.% Kupfer
5 bis 75 Gew.% Mangan
- B:: 0 bis 5 Gew.% Cobalt
25 bis 75 Gew.% Kupfer
20 bis 75 Gew.% Mangan
- C:: 75 bis 95 Gew.-% Cobalt
0 bis 5 Gew.-% Kupfer
5 bis 20 Gew.-% Mangan
Die im erfindungsgemäßen Verfahren verwendeten Katalysatoren werden im allgemeinen so hergestellt, daß man diese Metalle, beispielsweise als Hydroxide, Oxidhydrate, basische Salze oder Carbonate aus einer Lösung ihrer Salze, vorzugsweise ihrer Nitrate, Sulfate oder Acetate, durch Zugabe einer Base, vorteilhaft einer wäßrigen Mineralbase wie Natronlauge, Kalilauge, Natriumcarbonatlösung und/oder Kaliumcarbonatlösung gemeinsam fällt und den Niederschlag abtrennt, trocknet und calciniert. Vorzugsweise wird die Fällung nach dem Verfahren der DE-A 23 21 101 zweistufig durchgeführt, indem man zunächst die Metallsalzlösung mittels einer Alkalimetallcarbonatlösung bei Temperaturen von im allgemeinen 40 bis 70°C auf einen pH-Wert von wenigstens 8 bringt und danach zu dieser Fällungsmischung weitere Metallsalzlösung zudosiert bis sich ein pH-Wert von 6,8 bis 7,5 einstellt.

Der erhaltene Niederschlag wird nach üblichen Methoden, wie Filtration oder Zentrifugation, abgetrennt und gewaschen und anschließend getrocknet und calciniert. Die Calcinierung erfolgt im allgemeinen bei Temperaturen von 400 bis 550°C. Bei der Trocknung und Calcinierung wird der bei der Fällung erhaltene, chemisch heterogene Niederschlag der betreffenden schwerlöslichen Metallverbindungen in ein Konglomerat aus den Oxiden, Mischoxiden und gemischtvalentigen Oxiden dieser Metalle umgewandelt. Vor seiner Verwendung wird der auf diese Weise erhaltene Katalysator durch Reduktion mit Wasserstoff aktiviert, wobei die darin erhaltenen Metallverbindungen ganz oder teilweise zu den entsprechenden Metallen reduziert werden. Im allgemeinen wird diese Reduktion bei erhöhten Temperaturen, vorzugsweise bei 200 bis 600°C im Wasserstoffstrom vorgenommen. Diese Katalysatoraktivierung kann aber auch in situ, d.h. während des Verfahrens der Saccharosehydrierung erfolgen.

Katalysatoren mit besonders vorteilhaften mechanischen Eigenschaften können erhalten werden, indem man der Metallsalzlösung vor der Fällung anorganische Säuren, die zur Bildung von Polysäuren oder Heteropolysäuren befähigt sind, wie Schwefelsäure, Borsäure, Phosphorsäure, Molybdänsäure, Vanadinsäure, Wolframsäure und/oder deren Salze wie Trinatriumphosphat, Natriumtetraborat, Kaliumdihydrogenphosphat, Calciumhydrogenphosphat, Magnesiumhydrogenborat, Aluminiumphosphat, Natriummolybdat, Ammoniummolybdat, Ammoniumvanadat und/oder Natriumwolframat zusetzt und dann die Fällung und weitere Behandlung des Niederschlags wie beschrieben durchführt.

Alternativ zu dieser Vorgehensweise ist es möglich, diese Zusatzstoffe auf Mischungen der Oxide der katalytisch aktiven Metallkomponenten oder auf die calcinierte, ggf. vermahlene Katalysatormasse aufzutränken. Dazu vermengt man diese Feststoffe mit wäßrigen Lösungen der Salze der oben genannten Säuren und behandelt die erhaltene Mischung mit einer Mineralsäure, wie Salpeter- oder Schwefelsäure. Danach kann die derart modifizierte Katalysatormasse wie beschrieben oder gewünschtenfalls zu Katalysatorformkörpern geformt, getrocknet, calciniert und aktiviert werden. Die Menge der auf diese Weise in den Katalysator eingetragenen und darin verbleibenden Zusatzstoffe kann 0,1 bis 15 Gew.-%, bezogen auf den calcinierten Katalysator vor seiner Reduktion, betragen. Durch diese Behandlungsverfahren wird im allgemeinen die mechanische Handhabbarkeit des Katalysators verbessert, beispielsweise neigen auf diese Art hergestellte Katalysatorpulver weniger zum Verklumpen.

Nach der Calcinierung und vor ihrer Reduktion werden die Katalysatoren zweckmäßigerweise konditioniert, d.h. in diejenigen Applikationsformen gebracht, die für die von Betrieb zu Betrieb speziell konzipierten Produktionsanlagen am besten geeignet sind. So wird im Falle der kontinuierlichen Hydrierung von Saccharoselösungen vorzugsweise mit in der Sumpf- oder Rieselfahrweise betriebenen Festbettreaktoren gearbeitet, welche eine Katalysatorschüttung aus Katalysatorformkörpern oder Trägerkatalysatoren enthalten, wohingegen bei der diskontinuierlichen Betriebsweise die Anwendung eines im wäßrigen Reaktionsmediums suspendierten, pulverförmigen Katalysators bevorzugt ist.

Zur Herstellung der Suspensionskatalysatoren wird die calcinierte Katalysatormasse zweckmäßigerweise vor ihrer Reduktion im allgemeinen naß vermahlen und zwar bevorzugt auf eine mittlere Korngröße von 0,005 bis 0,5 mm.

Die pulverförmigen, aktivierten Suspensionskatalysatoren werden im allgemeinen in einer Menge von 30 bis 100, vorzugsweise 50 bis 70 g/kg einer 50 gew.-%igen wäßrigen Saccharoselösung verwendet. Werden höher oder niedriger konzentrierte Saccharoselösungen eingesetzt, so werden entsprechende Mengen an Katalysator der zu hydrierenden Lösung zugesetzt.

Trägerkatalysatoren werden im allgemeinen hergestellt, indem man die Katalysatorbestandteile nach dem beschriebenen Fällungsverfahren auf ein unter den Reaktionsbedingungen inertes Trägermaterial, das gewünschtenfalls in Form von Trägerformkörpern vorliegen kann, auffällt. Als Trägermaterialien können beispielsweise Aluminiumoxide, Siliciumdioxid, Zirkondioxid, Steatit oder Kaolin verwendet werden. Bevorzugt wird der Katalysator im vorliegenden Verfahren allerdings als Vollkatalysator, d.h. ohne den Zusatz einer Trägerkomponente, eingesetzt.

Mit den beschriebenen Katalysatoren lassen sich erfindungsgemäß wäßrige Saccharoselösungen zu Gemischen aus Diolen, Triolen und Tetrolen hydrieren.

Die Zusammensetzung der mit den beschriebenen Katalysatoren erhältlichen Hydrierprodukte ist von einer Reihe von Faktoren abhängig. So lassen sich besonders hohe Ausbeuten an Ethylenglykol und 1,2-Propylenglykol erzielen, wenn die Saccharosehydrierung bei Temperaturen von 230 bis 280°C durchgeführt wird, wohingegen bei Temperaturen von 180 bis 230°C im Hydrierprodukt höhere Anteile an Glycerin und 1,2,5,6-Hexantetrol gefunden werden. Bei Temperaturen oberhalb 280°C steigt der Gehalt an einwertigen Alkoholen im Hydrierprodukt an und bei Temperaturen unterhalb 180°C nimmt der Anteil an Hexiten wie Mannit oder Sorbit zu.

Die Saccharosehydrierung wird mit den vorliegenden Katalysatoren im allgemeinen mit Wasserstoffdrucken von 200 bis 700 bar, vorzugsweise bei 250 bis 300 bar, ausgeführt. Bei Drucken unterhalb 200 bar werden in zunehmenden Maße Hexite gebildet und oberhalb 700 bar wird die Saccharose in immer größerem Umfang zu einwertigen Alkoholen und sogar Kohlenwasserstoffen durchhydriert. Zwischen 200 und 700 bar Wasserstoffdruck ist die Zusammensetzung des Hydrierprodukts weitgehend vom angewandten Druck unabhängig.

Im erfindungsgemäßen Verfahren werden bevorzugt Saccharoselösungen mit einem Saccharosegehalt von 30 bis 60 Gew.-% hydriert. Die Hydrierung schwächer konzentrierter Saccharoselösungen ist im Hinblick auf die Zusammensetzung des Hydrierproduktes ebenfalls mit gutem Erfolg möglich, allerdings aufgrund der verringerten Raum-Zeit-Ausbeute wenig wirtschaftlich. Bei Saccharosekonzentrationen von mehr als 70 Gew.-% kann eine Selektivitätsabnahme hinsichtlich der entstehenden Hydrierprodukte beobachtet werden.

Es versteht sich von selbst, daß zur Erzielung einer hohen Selektivität auf eine gute Durchmischung von Saccharoselösung, suspendiertem Katalysator und Wasserstoffgas geachtet werden sollte. Eine solche läßt sich im Autoklaven beispielsweise durch die Anwendung eines Turbinenrührers sicherstellen.

Der Verlauf der Hydrierung läßt sich anhand des Wasserstoffverbrauches verfolgen. Wird nur noch wenig Wasserstoff von der Reaktionsmischung aufgenommen, so kann die Umsetzung beendet werden. Dieser Zeitpunkt wird im allgemeinen nach wenigen Stunden erreicht.

Bei Durchführung der Hydrierung im chargenweisen Betrieb wird im allgemeinen so vorgegangen, daß man den zweckmäßigerweise fein vermahlenen Katalysator im Autoklaven in Wasser suspendiert vorlegt. Zur besseren Suspendierung der Katalysatorpartikel kann es vorteilhaft sein, der wäßrigen Aufschlämmung noch 30 bis 80 Vol.-% der Polyole zuzusetzen, welche später bei der Saccharosehydrierung erzeugt werden sollen. Der Zusatz anderer Polyole ist zwar ebenfalls möglich, doch wird dadurch bei der Aufarbeitung des Reaktiongemisches die Isolierung der einzelnen Hydrierprodukte erschwert.

Mit den beschriebenen Katalysatoren wird die Saccharose im allgemeinen zügig, vollständig und mit guter Selektivität zu den gewünschten mehrwertigen Alkoholen hydriert. Bei der wiederholten Verwendung dieser Katalysatoren stellt man allerdings fest, daß zwar die Aktivität der Katalysatoren gleich bleibt, ihre Selektivität bezüglich der Bildung der technisch interessanten Diole, Triole und Tetrole aber von Hydriervorgang zu Hydriervorgang abnimmt, so daß nach etwa 5 bis 6 Hydriervorgängen (Zyklen) der Katalysator aufgrund ungenügender Selektivität unbrauchbar wird. Dieser Selektivitätsabfall macht sich insbesondere durch die Bildung einer Vielzahl von Nebenprodukten bemerkbar.

Es wurde nun gefunden, daß sich dieser Selektivitätsabfall erfindungsgemäß durch den Zusatz basisch wirkender Verbindungen zum Reaktionsmedium verhindern läßt. Infolgedessen kann der Katalysator beliebig oft wiederverwendet werden.

Als basisch wirkende Verbindungen können im erfindungsgemäßen Verfahren beispielsweise Alkalimetallhydroxide, Erdalkalimetallhydroxide, Alkalimetallcarbonate, Erdalkalimetallcarbonate, Stickstoffbasen wie Tetralkylammoniumhydroxide oder Carbonate oder aber auch Verbindungen verwendet werden, welche unter den Reaktionsbedingungen in basisch wirkende Verbindungen übergehen. Vorzugsweise werden die Hydroxide und Carbonate der Alkalimetalle und Erdalkalimetalle oder Mischungen dieser Verbindungen eingesetzt. Besonders förderlich wirken sich Zusätze der weniger gut löslichen Hydroxide und Carbonate der Metalle der ersten und zweiten Hauptgruppe sowie der Gruppe III A des Periodensystems auf die Selektivitätserhaltung des Katalysators aus. Besonders bevorzugt werden Lithiumhydroxid und/oder Magnesiumhydroxid dem Reaktionsmedium zugesetzt. Sehr gute Ergebnisse werden hinsichtlich der Selektivitätserhaltung beispielsweise auch mit einem Zusatz von Calciumoxid oder Natriumcarbonat erzielt. Beim Zusatz von Mischungen basisch wirkender Verbindungen zum Reaktionsmedium können sich die einzelnen Verbindungen bezüglich ihrer Wirksamkeit synergistisch beeinflussen. Beispielsweise führt der Zusatz von Kaliumoxid in Kombination mit Magnesiumoxid zu einem synergistischen Effekt.

Die basisch wirkenden Verbindungen werden im allgemeinen der zu hydrierenden Saccharoselösung in einer Menge von 0,01 bis 5 Gew.-%, vorzugsweise in einer Menge von 0,1 bis 1 Gew.-% und zweckmäßigerweise in fein verteilter Form oder als Lösung zugefügt. Höhere Zusätze dieser basisch wirkenden Verbindungen wirken sich zwar nicht nachteilig auf das Ergebnis der Saccharosehydrierung aus, erfordern aber im allgemeinen einen erhöhten Aufwand zu ihrer Abtrennung, weshalb zweckmäßigerweise auf höhere Zusätze verzichtet wird.

Mit den beschriebenen Katalysatoren können mit Hilfe des erfindungsgemäßen Zusatzes basisch wirkender Verbindungen und unter Anwendung der entsprechenden, beschriebenen Reaktionsbedingungen auf wirtschaftliche Weise Hydrierproduktgemische erhalten werden, welche wahlweise Diole oder Gemische aus Triolen und Tetrolen als Hauptprodukt enthalten.

Während die Diole - Ethylenglykol und 1,2-Propylenglykol - beispielsweise als Bestandteile von Frostschutzmitteln Verwendung finden, wird das 1,2,5,6-Hexantetrol zusammen mit Glycerin als vernetzende Alkoholkomponente bei der Herstellung von Polyurethan-Schaummassen benutzt.

### Beispiele

### Beispiel 1 (Vergleichsbeispiel)

In einem mit einem Begasungsrührer versehenen Autoklaven wurden 42 g des aktivierten Katalysators (mittlere Korngröße: 0,1 mm) in einer Lösung von 24 g Ethylenglykol und 56 g 1,2-Propylenglykol in 120 g Wasser suspendiert.

Es wurde ein Katalysator verwendet, der vor seiner Aktivierung mit Wasserstoff 450°C die folgende formale Zusammensetzung hatte:
66,8 Gew.% Cobalt, berechnet als CoO
19,2 Gew.% Kupfer, berechnet als CuO
7,1 Gew.% Mangan, berechnet als Mn₃O₄
3,4 Gew.% Phosphat, berechnet als H₃PO₄
3,5 Gew.% Molybdän, berechnet als MoO₃
Dies entspricht formal einem Cobalt-, Kupfer- und Mangangehalt von 72,21 bzw. 7 Gew.-% bezüglich des auf 100 Gew.-% normierten Gehaltes dieser drei Metalle im Katalysator.

Anschließend wurde durch Aufpressen von Wasserstoff ein Druck von 160 bar im Autoklaven erzeugt, der bei der Erhitzung der Reaktionsmischung auf die Reaktionstemperatur von 260°C auf 280 bar anstieg. Bei der Reaktionstemperatur wurden innerhalb einer Stunde 400 g einer 50 gew.-%igen wäßrigen Saccharoselösung in den Autoklaven gepumpt. Dabei blieb der Druck im Reaktionsgefäß in etwa konstant, da der durch die Volumenzunahme bedingte Druckanstieg im Reaktor durch den Verbrauch an Wasserstoff kompensiert wurde. Nach beendigter Saccharosezugabe wurde der Druck durch Nachpressen von Wasserstoff auf 300 bar eingestellt und während der Dauer der Hydrierung auf diesem Niveau gehalten.

Nach drei Stunden wurde die Hydrierung durch Abkühlen der Reaktionsmischung und Entspannen des Reaktionsgefäßes auf 5 bis 10 bar unterbrochen. Man ließ den Katalysator sedimentieren und entnahm die flüssige Reaktionsmischung dem Autoklaven über ein Steigrohr, so daß der Katalysator im Autoklaven verblieb.

Der so gewonnene Reaktionsaustrag enthielt 60 Gew.-% Wasser und 40 Gew.-% organische Bestandteile. Mittels Hochdruckflüssigchromatographie wurde für diese die folgende Zusammensetzung ermittelt:
65,5 Gew.% 1,2-Propylenglykol
19,5 Gew.% Ethylenglykol
4,9 Gew.% 1,2-Butylenglykol
3,6 Gew.% Hexan-1,2,5,6-tetrol
0,8 Gew.% Hexan-1,2,6-triol
3,0 Gew.% einwertige Alkohole wie Ethanol, n-Propanol, 2-Butanol
2,7 Gew.% nicht identifizierte Verbindungen
Der Hydriervorgang wurde auf diese Weise mit demselben Katalysator mehrmals wiederholt, um die Lebendauer des Katalysators in Katalysatorzyklen zu bestimmen. Dabei wurde festgestellt, daß bei Zugabe reiner Saccharoselösung der Propylenglykolanteil im Reaktionsaustrag mit zunehmender Zyklenzahl linear abfiel.

Nach dem sechsten Hydriervorgang enthielt der Austrag 62,3 Gew.-% Wasser und 37,7 Gew.-% organische Bestandteile, mit folgender Zusammensetzung:
30,7 Gew.% 1,2-Propylenglykol
10,7 Gew.% Ethylenglykol
9,9 Gew.% 1,2-Butylenglykol
13,8 Gew.% Hexan-1,2,5,6-tetrol
11,2 Gew.% Hexan-1,2,6-triol
5,5 Gew.% einwertige Alkohole
18,2 Gew.% nicht identifizierte Verbindungen.

### Beispiel 2

Die Hydrierung wurde wie in Beispiel 1 beschrieben durchgeführt. Es wurden jedoch in 400 g zu hydrierender Saccharoselösung 1 g Lithiumhydroxid fein verteilt suspendiert. Nach 18 Katalysatorzyklen waren noch 90 % des Gehaltes an 1,2-Propylenglykol, verglichen mit dem Ergebnis des ersten Hydriervorganges, im Reaktionsaustrag enthalten.

### Beispiel 3

Die Hydrierung wurde wie in Beispiel 1 beschrieben durchgeführt. Als basisch wirkende Verbindung wurde 1 g Magnesiumhydroxid fein verteilt in 400 g wäßriger Saccharoselösung suspendiert. Nach 9 Zyklen waren noch 95 % des Gehaltes an 1,2-Propylenglykol, verglichen mit dem Ergebnis des ersten Hydriervorganges, im Reaktionsaustrag enthalten.

### Beispiel 4

Die Hydrierung wurde wie in Beispiel 1 beschrieben durchgeführt. In 400 g Saccharoselösung wurden jedoch 2 g Calciumoxid fein verteilt suspendiert. Nach 19 Zyklen waren noch 91 % des Gehaltes an 1,2-Propylenglykol, verglichen mit dem Ergebnis des ersten Hydriervorganges, im Reaktionsaustrag enthalten.

### Beispiel 5

Die Hydrierung wurde wie in Beispiel 1 durchgeführt. In 400 g wäßriger Saccharoselösung wurde 1 g Magnesiumoxid, das 4 Gew.-% Kaliumoxid enthielt, fein verteilt suspendiert. Nach 36 Zyklen waren noch 89 % des Gehaltes an 1,2-Propylenglykol, verglichen mit dem Ergebnis des ersten Hydriervorganges, im Reaktionsaustrag enthalten.

### Beispiel 6

In einem Autoklaven von 10 l Inhalt wurden 370 g des aktivierten Katalysators (mittlere Korngröße: 0,1 mm) sowie 20 g Magnesiumoxid, das 2 Gew.-% Kaliumoxid enthielt, in 5 700 g einer 50 gew.-%igen wäßrigen Saccharoselösung suspendiert. Es wurde ein Katalysator mit der gleichen chemischen Zusammensetzung wie im Beispiel 1 beschrieben verwendet.

Anschließend wurde durch Aufpressen von Wasserstoff ein Druck von 180 bar im Autoklaven erzeugt und die Reaktionsmischung unter intensivem Rühren innerhalb von 2 Stunden auf 215°C erhitzt. Bei 180°C zeigte ein stark ansteigender Wasserstoffverbrauch das Einsetzen der Reaktion an. Der durch die Wasserstoffaufnahme verursachte Druckabfall wurde laufend durch Nachpressen frischen Wasserstoffs ausgeglichen und der Druck im Autoklaven auf diese Weise zwischen 280 und 300 bar gehalten.

Nach 4,5 Stunden wurde die Hydrierung durch Abkühlen und Entspannen des Autoklaven beendet. Der flüssige Reaktionsaustrag wurde wie in Beispiel 1 beschrieben vom Katalysator abgetrennt und der Hydriervorgang wie beschrieben mehrfach wiederholt. Dabei konnte festgestellt werden, daß sich der Katalysator unter den angewandten Reaktionsbedingungen beliebig oft, ohne Nachlassen seiner Wirksamkeit verwenden ließ.

Der auf diese Weise gewonnene Reaktionsaustrag enthielt 55 Gew.-% Wasser und 45 Gew.-% organische Bestandteile, mit folgender Zusammensetzung:
30 Gew.-% Hexan-1,2,5,6-Tetrol
18 Gew.-% Glycerin
23,3 Gew.-% 1,2-Propylenglykol
11,2 Gew.-% Ethylenglykol
3 Gew.-% Butylenglykol
7 Gew.-% Hexan-1,2,6-Triol
0,5 Gew.-% einwertige Alkohole
7 Gew.-% nichtidentifizierte Verbindungen
Zur Isolierung der organischen Bestandteile wurde zunächst das Wasser aus dem Reaktionsaustrag bei vermindertem Druck abgedampft. Aus dem Abdampfrückstand wurden Ethylenglykol, Propylenglykol, Glycerin und Butylenglykol durch fraktionierte Destillation bei vermindertem Druck isoliert. Der verbleibende viskose Rückstand wurde durch eine Hochvakuumdestillation in einem Dünnschichtverdampfer weiter gereinigt.

## Patentansprüche

1. Verfahren zur Herstellung niederer, mehrwertiger Alkohole durch die katalytischer Hydrierung wäßriger Saccharoselösungen bei erhöhter Temperatur und bei erhöhtem Druck und mittels eines Katalysators, dessen katalytisch aktive Masse im wesentlichen die Metalle Cobalt, Kupfer und Mangan in Mengen von, berechnet auf den auf 100 % normierten Gesamtgehalt an diesen Metallen,
0 bis 100 Gew.% Cobalt
0 bis 85 Gew.% Kupfer und
0 bis 80 Gew.% Mangan
enthält, wobei diese katalytisch aktive Masse von den genannten Metallen entweder nur Cobalt oder mindestens zwei der genannten Metalle enthält und dem außerdem noch anorganische Polysäuren und/oder Heteropolysäuren zugesetzt sein können, dadurch gekennzeichnet, daß man der zu hydrierenden Saccharoselösung 0,01 bis 5 Gew.-% einer oder mehrerer basisch wirkender Metallverbindungen der Metalle der ersten und zweiten Hauptgruppe sowie der Gruppe III A des Periodensystems zusetzt.

2. Verfahren nach Anspruch 1, dadurch gekennzeichnet, daß man der zu hydrierenden Saccharoselösung 0,1 bis 1 Gew.-% einer oder mehrerer basisch wirkender Metallverbindungen der Metalle der ersten und zweiten Hauptgruppe sowie der Gruppe III A des Periodensystems zusetzt.

3. Verfahren nach Anspruch 1, dadurch gekennzeichnet, daß man als basisch wirkende Verbindungen basische Alkalimetall- und/oder Erdalkalimetallverbindungen benutzt.

4. Verfahren nach Anspruch 1, dadurch gekennzeichnet, daß man als basisch wirkende Verbindungen die Hydroxide, Oxide und/oder Carbonate des Lithiums, Natriums, Kaliums, Magnesiums und/oder Calciums benutzt.

5. Verfahren nach Anspruch 1, dadurch gekennzeichnet, daß man die Hydrierung bei Temperaturen von 230 bis 280°C durchführt.

6. Verfahren nach Anspruch 1, dadurch gekennzeichnet, daß man die Hydrierung bei Temperaturen von 180 bis 230°C durchführt.

## Claims

1. A process for the preparation of lower polyhydric alcohols by catalytic hydrogenation of aqueous sucrose solutions at elevated temperature and superatmospheric pressure and by means of a catalyst whose catalytically active material essentially comprises the metals cobalt, copper and manganese in amounts of, based on the total content of these metals standardized to 100%,
from 0 to 100% by weight of cobalt,
from 0 to 85% by weight of copper and
from 0 to 80% by weight of manganese,
where this catalytically active material contains, of the said metals, either only cobalt or at least two of said metals, and to which, in addition, inorganic polyacids and/or heteropolyacids may be added, which comprises adding from 0.01 to 5% by weight of one or more basic metal compounds of metals from the first and second main group and from group III A of the Periodic Table to the sucrose solution to be hydrogenated.

2. A process as claimed in claim 1, wherein from 0.1 to 1% by weight of one or more basic metal compounds of metals from the first and second main group and from group III A of the Periodic Table is added to the sucrose solution to be hydrogenated.

3. A process as claimed in claim 1, wherein the basic compounds used are basic alkali metal and/or alkaline earth metal compounds.

4. A process as claimed in claim 1, wherein the basic compounds used are the hydroxides, oxides and/or carbonates of lithium, sodium, potassium, magnesium and/or calcium.

5. A process as claimed in claim 1, wherein the hydrogenation is carried out at from 230 to 280°C.

6. A process as claimed in claim 1, wherein the hydrogenation is carried out at from 180 to 230°C.

## Revendications

1. Procédé de préparation de polyalcools inférieurs par l'hydrogénation catalytique de solutions aqueuses de saccharose à température élevée, sous pression élevée et au moyen d'un catalyseur dont la masse catalytiquement active contient essentiellement les métaux cobalt, cuivre et manganèse, dans des proportions, calculées sur la base de la teneur totale en ces métaux normalisée à 100%, de
0 à 100% en poids de cobalt,
0 à 85% en poids de cuivre et
0 à 80% en poids de manganèse,
cette masse catalytiquement active contenant, parmi lesdits métaux, soit du cobalt seulement, soit deux au moins desdits métaux et, en outre, des polyacides et/ou des hétéropolyacides inorganiques pouvant encore lui être ajoutés, caractérisé en ce qu'on ajoute, à la solution de saccharose à hydrogéner, de 0,01 à 5% en poids d'un ou de plusieurs composés métalliques à réaction basique des métaux des groupes IA et IIA, ainsi que du groupe IIIA de la classification périodique.

2. Procédé selon la revendication 1, caractérisé en ce qu'on ajoute, à la solution de saccharose à hydrogéner, de 0,1 à 1% en poids d'un ou de plusieurs composés métalliques à réaction basique des métaux des groupes IA et IB, ainsi que du groupe IIIA de la classification périodique.

3. Procédé selon la revendication 1, caractérisé en ce qu'on utilise, comme composés à réaction basique, des composés de métaux alcalins et/ou de métaux alcalino-terreux.

4. Procédé selon la revendication 1, caractérisé en ce qu'on utilise, comme composés à réaction basique, les hydroxydes, oxydes et/ou carbonates du lithium, du sodium, du potassium, du magnésium et/ou du calcium.

5. Procédé selon la revendication 1, caractérisé en ce qu'on conduit l'hydrogénation à des températures de 230 à 280°C.

6. Procédé selon la revendication 1, caractérisé en ce qu'on conduit l'hydrogénation à des températures de 180 à 230°C.
